(19) **Europäisches Patentamt European Patent Office Office européen des brevets**

(11) **EP 4 628 009 A1**

(12) **EUROPEAN PATENT APPLICATION**

(43) Date of publication:
08.10.2025 Bulletin 2025/41

(21) Application number: 24461556.3

(22) Date of filing: 04.04.2024

(51) International Patent Classification (IPC):
*A61B 5/087* (2006.01)  *A61B 5/091* (2006.01)
*A61B 5/097* (2006.01)  *A61B 5/00* (2006.01)

(52) Cooperative Patent Classification (CPC):
**A61B 5/087; A61B 5/091; A61B 5/097;**
**A61B 5/7203**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Findair Sp. z o. o.**
**31-235 Krakow (PL)**

(72) Inventors:
• **MIKOSZ, Jacek**
 **31-162 Krakow (PL)**
• **CZYZ, Michal**
 **30-719 Krakow (PL)**
• **MIKOSZ, Tomasz**
 **32-091 Michalowice (PL)**

(74) Representative: **Kancelaria Eupatent.pl Sp. z o.o.**
**ul. Kilinskiego 185**
**90-348 Lodz (PL)**

(54) **A VERSATILE AIRFLOW SENSOR**

(57)  An airflow sensor (10) comprising: a housing (11) with mounting means (12) for attaching the airflow sensor (10) to a tube (31, 41, 51) within which airflow is to be measured; at least one measurement microphone (15A) for capturing audio signals of airflow within the tube to which the housing (11) is connected; and a controller (20) with a data converter (21) module for converting audio data collected by the measurement microphone (15A) into data representative of the airflow, wherein the converter (21) is configured to convert data according to at least two of the following modes of operation: a PMDI inhaler mode, a spirometer mode, a peak flow meter mode, a mouth-nasal flow mode.

**Fig. 1A**

EP 4 628 009 A1

**Description**

TECHNICAL FIELD

The present invention pertains to an airflow sensor designed for use with air intake devices such as inhalers, spirometers, and peak flow meters.

BACKGROUND

Airflow sensors are widely used in various medical devices such as inhalers, spirometers, and peak flow meters. These sensors are designed to measure the rate of airflow and are critical in monitoring and managing respiratory conditions. Traditional airflow sensors are typically designed for a specific type of device and are not versatile in their application.

Inhalers, for instance, require sensors that can accurately measure the force and timing of inhalation to ensure effective delivery of medication. Spirometers, on the other hand, require sensors that can measure lung capacity and the speed of inhalation and exhalation. Peak flow meters, used primarily by asthma patients, require sensors that can measure the maximum speed of expiration.

While these devices serve different purposes, they all rely on the accurate measurement of airflow. However, the lack of versatility in traditional airflow sensors means that a different sensor is required for each type of device. This not only increases the cost of these devices but also limits their functionality.

SUMMARY OF THE INVENTION

Therefore, there is a need to provide a more versatile airflow sensor which would at least partially solve at least some of the problems existing in the prior art solutions.

An airflow sensor may be understood as a device designed to measure the flow of air through a specific medium, often used in medical applications to monitor and analyze air movement. This invention specifically addresses the need for a versatile airflow sensor capable of being adapted for use with various air intake devices such as inhalers, spirometers, and peak flow meters, thereby offering a solution to the limitations presented by traditional airflow sensors which are typically designed for a singular purpose.

The airflow sensor comprises a housing with mounting means for attaching the airflow sensor to a tube within which airflow is to be measured. The housing serves as the structural framework of the sensor, protecting internal components and providing a means to secure the sensor to different devices. The mounting means, such as guide rails or other attachment mechanisms, enable the sensor to be easily and securely connected to various tubes, ensuring that the sensor can be utilized across multiple applications. One advantage of this arrangement is the enhanced versatility and utility of the airflow sensor, as it can be adapted for use with different types of air intake devices without the need for multiple, device-specific sensors. This not only reduces the cost associated with acquiring and maintaining different sensors for each device but also simplifies the process of monitoring and analyzing airflow in various contexts, leading to better use of processing power and storage capacity.

Furthermore, the airflow sensor includes at least one measurement microphone for capturing audio signals of airflow within the tube to which the housing is connected. The measurement microphone detects the sound generated by air moving through the tube. This sound is then converted into data representative of the airflow, allowing for accurate measurement and analysis. The technical advantage of including at least one measurement microphone lies in the sensor's ability to capture detailed audio signals, which, when processed, provide precise and reliable measurements of airflow. This precision is crucial in medical applications where accurate dosing and monitoring of respiratory activities are essential for effective treatment and management of health conditions.

Additionally, the sensor is equipped with a controller that includes a data converter module. This module is responsible for converting the audio data collected by the measurement microphone into data representative of the airflow. The converter can be configured to operate in at least two modes, such as a PMDI inhaler mode, a spirometer mode, a peak flow meter mode, and a mouth-nasal flow mode, allowing the sensor to adapt its processing according to the specific application it is being used for. The inclusion of a versatile data converter module offers the technical advantage of enabling the sensor to accurately interpret audio signals across different modes of operation, ensuring that the airflow measurements are accurate and relevant to the specific device and context in which the sensor is used. This adaptability enhances the sensor's utility in a wide range of applications, from patient education and inhalation therapy to pulmonary rehabilitation and spirometry, making it a valuable tool in respiratory medicine. The use of Fast Fourier Transform (FFT) analysis in the data conversion process further underscores the sensor's capability to provide accurate and detailed measurements of airflow by analyzing the frequency components of the captured audio signals.

The airflow sensor may further comprise a display. The inclusion of a display offers the advantage of immediate feedback to the user, presenting measurement results and other relevant information in an easily accessible manner. This feature is particularly useful in clinical settings and for patient education, where real-time data can significantly impact the understanding and management of respiratory conditions.

The sensor may also include communication means for data transfer, expanding its functionality by enabling the sharing of collected data with other devices or systems. This capability is crucial for integrating the sensor into broader health monitoring frameworks, facilitating remote patient monitoring, and enhancing the collaborative efforts of healthcare teams.

A distance sensor for detecting whether the airflow sensor has been attached to a tube is another feature that may be provided. This sensor ensures the correct positioning of the airflow sensor, which is essential for accurate measurements. The ability to confirm attachment before operation helps prevent user error and enhances the reliability of the data collected.

Moreover, the airflow sensor may comprise one or more buttons for controlling the operation of the sensor. This feature introduces a user-friendly interface, allowing for straightforward operation and interaction with the sensor, which is beneficial in both clinical and home settings.

Furthermore, the sensor may comprise a cancellation microphone for collecting ambient sound signals, wherein the data converter module is configured to process audio data collected by the measurement microphone reduced by the audio data collected by the cancellation microphone, addresses the challenge of ambient noise interference. This arrangement significantly improves the accuracy of airflow measurements by ensuring that only the sounds directly related to airflow are analyzed, thereby enhancing the sensor's performance in various environments.

In summary, the present invention introduces a versatile and adaptable airflow sensor that addresses the limitations of traditional, device-specific sensors. Through its innovative design, including a housing with adaptable mounting means, a measurement microphone for capturing detailed audio signals, and a controller with a versatile data converter module, the invention offers significant advantages in terms of versatility, accuracy, and utility across various applications in respiratory medicine.

The airflow sensor according to the invention provides the possibility of being attached to various adapters that extend the sensor's functionality while still utilizing its original premise of converting sound profiles to airflow in liters. The invention has applications in various areas of respiratory medicine, from educating patients with asthma or chronic obstructive pulmonary disease (COPD) to pulmonary rehabilitation and spirometry. The key feature of this invention is its multifunctionality and flexibility. By using a versatile method of converting sound to airflow, with the addition of a proper airflow conditioning adapter, it is possible to both learn proper inhalation and perform breathing exercises and simple lung function tests.

## BRIEF DESCRIPTION OF DRAWINGS

The present invention is shown by means of example embodiments in a drawing, wherein:

Figs. 1A and 1B show an overall view of the airflow sensor from the front and back, respectively;

Fig. 2 shows a functional diagram of the airflow sensor;

Figs. 3A and 3B show an adapter for a PMDI inhaler and the airflow sensor connected to the PMDI inhaler, respectively;

Figs. 4A and 4B show a tube and the airflow sensor connected to the tube to function as a spirometer, respectively;

Figs. 5A and 5B show a face mask with a tube and the airflow sensor connected to the tube to function as a mouth-nose flow meter, respectively;

## DETAILED DESCRIPTION

The following detailed description is of the best currently contemplated modes of carrying out the invention. The description is not to be taken in a limiting sense, but is made merely for the purpose of illustrating the general principles of the invention.

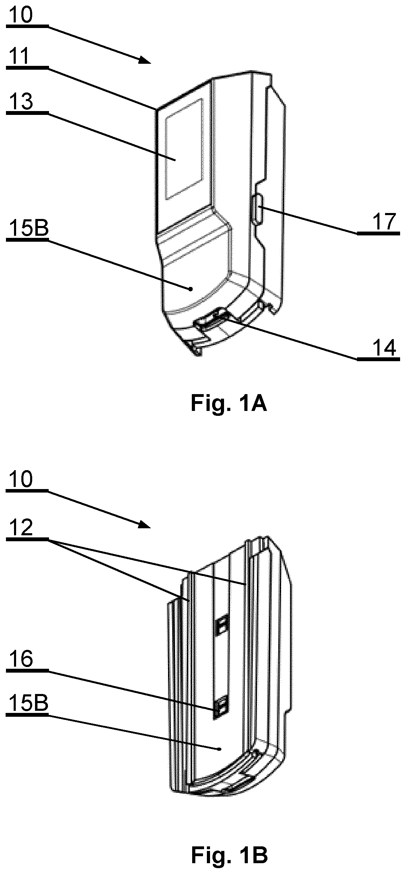

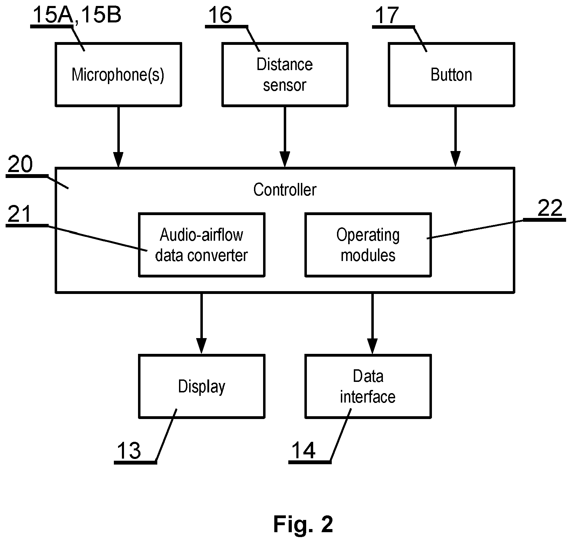

The airflow sensor 10, as shown in Figs. 1A, 1B, and Fig. 2, is designed as a measurement module. It features a box-shaped housing 11 with mounting means 12, such as guide rails, located on its back wall for attaching the airflow sensor 10 to tubes within which airflow is to be measured. A display 13, such as an LCD screen, is located on the front wall for displaying the status of the sensor 10 and other relevant information, such as measurement results, battery level, etc. A connector port 14, which can be located on the bottom wall, allows for the attachment of a charging cable, such as a USB connector, for charging the internal battery of the sensor 10 and optionally for data transfer (although data transfer can also be achieved via a wireless connector). At least one measurement microphone 15A is positioned such as to be in proximity to the tube to which the sensor 10 is to be attached, preferably near the mounting rails, for capturing audio signals to be converted into airflow data. In addition, a compensation microphone 15B is positioned away from the tube, such as to measure ambient noise for noise compensation. The sensor 10 may also include a distance sensor 16 for detecting whether the sensor has been attached to an airflow tube. One or more buttons 17 may be provided for controlling the operation of the sensor 10, such as for starting and stopping the measurement.

Data from the microphones 15A, 15B, the distance sensor 16, and the button 17 is input into a controller 20 mounted within the housing 11. The controller 20 can be any suitable type of microprocessor with memory or a system-on-chip circuit. The controller 20 includes a data converter 21 module for converting audio data collected by the measurement microphone 15A into airflow data. The controller 20 also includes other operating modules 22 for coordinating the operation of the sensor 10, such as starting/stopping operation, preparing data for presentation on the display 13, and preparing data for transmission via the data interface 14 (such as the USB port or any other wired or wireless interface, such as WiFi or Bluetooth).

The measurement microphone 15A is used for detecting the sound of airflow during the use of the device to which the sensor is connected. The audio data collected by the measurement microphone 15A is then converted by the converter 21 into respiratory parameters. In case a cancellation microphone 15B is used, the converter 21 may process a signal corresponding to a difference between the signal of the measurement microphone 15A and the cancellation microphone 15B, such as to cancel the ambient sounds from the signal measured from the tube. In case there are more measurement microphones 15A, the converter 21 may be configured to process a signal of a selected one of the measurement microphones 15A (for example, the one that is closest to the tube, depending on the type of tube to which the sensor 10 is connected), or the strongest of all of the signals or an average of all of the signals.

The conversion is performed according to a mode of operation, wherein the modes of operation may include:

- for a PMDI inhaler mode: Peak Inspiratory Flow Rate (PIFR), time of inhalation, total inhalation volume;
- for a spirometer mode: Peak Expiratory Flow (PEF), total exhalation volume;
- for a peak flow meter mode: Peak Expiratory Flow (PEF), total exhalation volume;
- for a mouth-nasal flow mode: number of inhalation-exhalation occurrences during session, inhalation session time, PIFR.

The converter 21 operates according to an algorithm for converting sound into airflow, which enables effective analysis and recording of critical aspects of breathing. Thus, the module offers detailed measurement and monitoring of breathing, which is important in respiratory and inhalation therapies. Preferably, the algorithm is based on FFT (Fast Fourier Transform) analysis. The sound processing method uses the Fast Fourier Transform (FFT) algorithm to spectrally analyze the sound generated when air flows through the inhaler. In this process, the sound signal, originally in the time domain, is converted to the frequency domain. Then, the algorithm calculates the average power for all frequencies in this transformed signal. These values are then logarithmically transformed into airflow expressed in liters, which allows precise determination of respiratory parameters, such as strength and inhalation time. The use of FFT enables detailed analysis of acoustic characteristics of airflow, which is crucial for monitoring and optimizing inhalation techniques.

For example, the audio data from the measurement microphone 15A can be sampled with a 32kHz frequency. The sampled data can be gathered to series of a length of 1/16 second, i.e. 2000 samples in each series. The series are then subject to FFT to obtain sound level in 128 frequency ranges. Next, an average level of all values is calculated. The average level (avg) is used to calculate flow based on the following formula:

$$flow = a * log(avg + b) + c * avg + d,$$

wherein a, b, c, d are parameters specific to the geometry of the particular sensor and the particular tube to which it is attached and can be predefined upon configuration of the sensor. The result of the computation - the value of "flow" parameter - indicates the amount of air (e.g. in liters) transmitted via the site of measurement in a unit of time. For example, it can be used to indicate the flow in liters per minute. Based on the value of the flow parameter, further parameters can be determined, such as PIFR (by determining the moment of the highest flow during the inspiration phase) or PEF (by determining the moment of the highest flow during the expiration phase).

In the embodiment presented in Fig. 1B, the mounting means 12 are in the form of guide rails. However, various other mounting means 12 can be used.

Using the mounting means 12, the sensor 10 can be connected to various tubes to measure airflow through them.

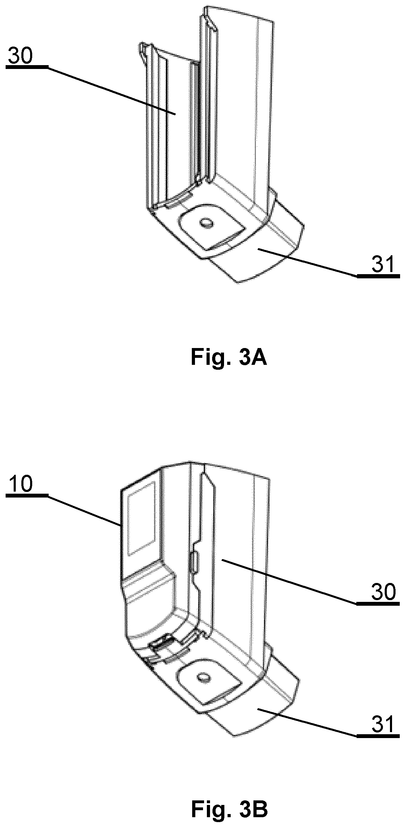

For example, as shown in Figs. 3A and 3B, the sensor 10 can be connected to an adapter 30 that is coupled with a PMDI-type inhaler 31. In this configuration, the sensor 10 can help the user to learn the correct method of inhalation. It can measure the force of inhalation, the timing of drug release relative to inhalation, and the shaking of the inhaler. This functionality is especially useful for patients with lung diseases who need to use inhalation regularly and skillfully coordinate inhalation with drug release. Thanks to the measurement capabilities, patients can be educated about the correct inhalation technique, which is crucial to the effectiveness of therapy. The sensor 10 can serve as a training tool, helping patients improve their inhalation technique, which is important in optimizing drug delivery to the lungs.

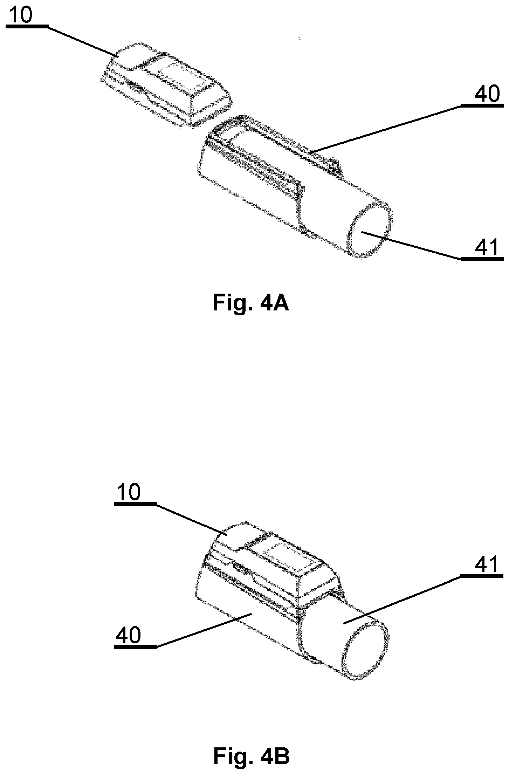

Furthermore, as shown in Figs. 4A and 4B, the sensor 10 can be connected to an adapter 40 that is coupled with a tube 41 to form a spirometer or a peak flow meter. In this configuration, the sensor 10 can measure respiratory parameters of the user, such as lung capacity and airflow.

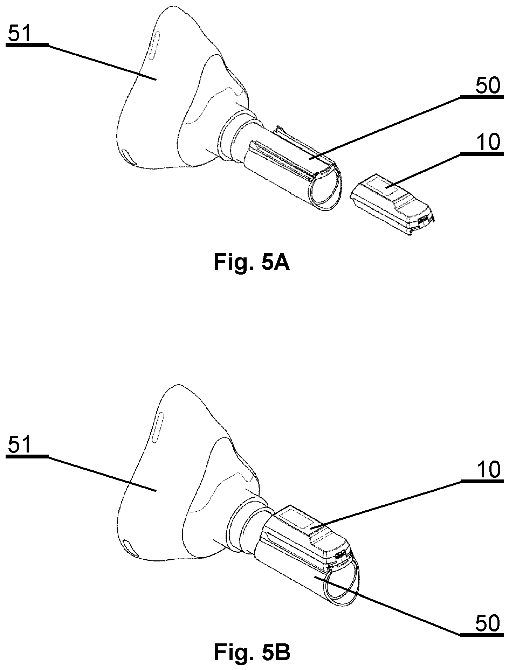

In yet another embodiment, as shown in Figs. 5A and 5B, the sensor 10 can be connected to an adapter 50 that is coupled with a breathing mask 51 to measure mouth-nose airflow parameters or encourage the user to perform breathing games. Breathing games can be used to perform breathing exercises, which are important in pulmonary rehabilitation and lung training.

While the invention has been described with respect to a limited number of embodiments, it will be appreciated that many variations, modifications and other applications of the invention may be made. Therefore, the claimed invention as recited in the claims that follow is not limited to the embodiments described herein.

**Claims**

1. An airflow sensor (10) comprising:

   - a housing (11) with mounting means (12) for attaching the airflow sensor (10) to a tube (31, 41, 51) within which airflow is to be measured;
   - at least one measurement microphone (15A) for capturing audio signals of airflow within the tube to which the housing (11) is connected; and
   - a controller (20) with a data converter (21) module for converting audio data collected by the measurement microphone (15A) into data representative of the airflow, wherein the converter (21) is configured to convert data according to at least two of the following modes of operation: a PMDI inhaler mode, a spirometer mode, a peak flow meter mode, a mouth-nasal flow mode.

2. The airflow sensor according to claim 1, further comprising a display (13).

3. The airflow sensor according to any of previous claims, further comprising communication means (14) for data transfer.

4. The airflow sensor according to any of previous claims, further comprising a distance sensor (16) for detecting whether the airflow sensor (10) has been attached to a tube.

5. The airflow sensor according to any of previous claims, further comprising one or more buttons (17) for controlling the operation of the airflow sensor (10).

6. The airflow sensor according to any of previous claims, wherein the mounting means (12) are guide rails.

7. The airflow sensor according to any of previous claims, wherein the data converter (21) module is configured to operate based on FFT (Fast Fourier Transform) analysis.

8. The airflow sensor according to any of previous claims, further comprising a cancellation microphone (15B) for collecting ambient sound signals, wherein the data converter (21) module is configured to process audio data collected by the measurement microphone (15A) reduced by the audio data collected by the cancellation microphone (15B).

10

11

13

15B

17

14

**Fig. 1A**

10

12

16

15B

**Fig. 1B**

**15A,15B** Microphone(s)

**16** Distance sensor

**17** Button

**20** Controller

**21** Audio-airflow data converter

**22** Operating modules

**13** Display

**14** Data interface

**Fig. 2**

30

31

**Fig. 3A**

10

30

31

**Fig. 3B**

**Fig. 4A**

**Fig. 4B**

51

50

10

**Fig. 5A**

51

10

50

**Fig. 5B**

Europäisches
Patentamt

European
Patent Office

Office européen
des brevets

# EUROPEAN SEARCH REPORT

**Application Number**

EP 24 46 1556

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/317379 A1 (BRIMER ANDREW [US] ET AL) 28 November 2013 (2013-11-28) <br> * paragraphs [0005], [0008], [0067], [0082] * <br> * paragraphs [0103], [0106], [0119] * <br> * figures 19-22, 26, 32 * | 1-8 | INV. <br> A61B5/087 <br> A61B5/091 <br> A61B5/097 <br> A61B5/00 |
| X | WO 2023/201090 A1 (HUMAN RESOLUTION TECH LLC [US]) 19 October 2023 (2023-10-19) <br> * paragraphs [0004], [0053] - [0058], [0075] * <br> * paragraphs [0076], [0084], [0088], [0098] * <br> * figures 2B, 3, 4A * | 1-8 | |
| A | US 2013/190641 A1 (GONNEN LIOR [IL] ET AL) 25 July 2013 (2013-07-25) <br> * figures 1, 2d, 2e * <br> * paragraphs [0009] - [0014] * <br> * paragraphs [0032] - [0037], [0046] * | 1-8 | |

**TECHNICAL FIELDS SEARCHED (IPC)**

A61B

The present search report has been drawn up for all claims

| Place of search | Date of completion of the search | Examiner |
|---|---|---|
| The Hague | 13 May 2024 | Athanasiadis, I |

EPO FORM 1503 03.82 (P04C01)

## ANNEX TO THE EUROPEAN SEARCH REPORT
## ON EUROPEAN PATENT APPLICATION NO.

EP 24 46 1556

This annex lists the patent family members relating to the patent documents cited in the above-mentioned European search report.
The members are as contained in the European Patent Office EDP file on
The European Patent Office is in no way liable for these particulars which are merely given for the purpose of information.

13-05-2024

| Patent document cited in search report | | Publication date | Patent family member(s) | | Publication date |
|---|---|---|---|---|---|
| US 2013317379 | A1 | 28-11-2013 | AU | 2013266376 A1 | 15-01-2015 |
| | | | AU | 2017228552 A1 | 05-10-2017 |
| | | | CA | 2874447 A1 | 28-11-2013 |
| | | | CN | 104519795 A | 15-04-2015 |
| | | | CN | 107307868 A | 03-11-2017 |
| | | | EP | 2852322 A1 | 01-04-2015 |
| | | | US | 2013317379 A1 | 28-11-2013 |
| | | | US | 2017265776 A1 | 21-09-2017 |
| | | | WO | 2013177300 A1 | 28-11-2013 |
| WO 2023201090 | A1 | 19-10-2023 | NONE | | |
| US 2013190641 | A1 | 25-07-2013 | US | 2013190641 A1 | 25-07-2013 |
| | | | WO | 2012038903 A2 | 29-03-2012 |

EPO FORM P0459

For more details about this annex : see Official Journal of the European Patent Office, No. 12/82